# EUROPEAN PATENT SPECIFICATION

(11) **EP 2 522 997 B1**
(45) Date of publication and mention of the grant of the patent: **29.01.2014**
(21) Application number: 11166092.4
(22) Date of filing: 13.05.2011
(51) Int. Cl.: G01N 33/28, E21B 33/035, E21B 34/04, E21B 43/12

(54) **Monitoring hydrocarbon fluid flow**
Überwachung eines Kohlenwasserstoffflüssigkeitsflusses
Monitoring hydrocarbon fluid flow

(43) Date of publication of application: 14.11.2012
(73) Proprietor: Vetco Gray Controls Limited, Bristol BS48 1BS (GB)
(72) Inventor: Ellson, Nicholas, Nailsea, Bristol BS48 1BS (GB); Vyas, Parag, D-85748 Garching bei München (DE); Phillips, Raymond, Nailsea, Bristol BS48 1BS (GB); Busboom, Axel, D-85748 Garching bei München (DE)
(74) Representative: Emerson, Peter James

(56) References cited:
- WO-A1-2009/136950
- GB-A- 2 456 231
- US-A1- 2008 257 032
- US-A1- 2010 051 286

## Description

### Field of the Invention

The present invention relates to monitoring hydrocarbon fluid flow, more particularly at a tree of a subsea hydrocarbon extraction facility.

### Background of the Invention

Hydrocarbon fluid flowing from an offshore reservoir or well is multiphase in nature in that it contains oil, gas and water and can also contain particulates such as sand. Multiphase meters are used to measure the content of gas, oil and water in the fluid and other sensors are incorporated to measure the particulates. The sensor equipment is normally mounted on the Christmas tree installed on the seabed and is usually placed on the Christmas tree after the design has been established and therefore the sensor location is often dictated by practical issues rather than the optimum position for measurements. Current practice is to install a multiphase meter on the Christmas tree with a sensor package positioned at a convenient position. The sensor package usually contains a bundle of sensors and so the individual sensors may not all be ideally positioned to accurately measure their particular parameter. Some fluid flow measurement techniques require the flow to be conditioned (for example, laminar or turbulent) to be at their most accurate. There is a need for a more accurate method of measurement.

One of the most critical aspects of fluid flow is the effect that it has on equipment and fluid pipes due to:
- hydrate formation
- wax deposition
- slugging
- corrosion

These have a serious effect on efficient fluid flow, equipment lifetime and through-life maintenance requirements, but can be minimised by taking appropriate remedial action such as the use of chemical injection to clean the surfaces of the fluid pipes. There is a need for accurately identifying, locating and measuring these effects. However, the availability of sufficiently reliable and accurate sensors has limited the capability to provide the required information and also because the positioning of the sensor package on the Christmas tree is decided after the Christmas tree design has been established and so the positioning is not optimised.

GB-A-2 456 231 discloses a method having the pre-characterising features of claim 1.

WO 2009/136950 discloses a method for monitoring a Christmas tree assembly installed on a subsea hydrocarbon well which includes receiving a plurality of parameters associated with the assembly. US 2008/0257032 discloses a structure adapted to be removably coupled to a Christmas tree, a sleeve operatively coupled to the structure and a flow meter positioned at least partially within the sleeve.

### Summary of the Invention

According to the present invention from one aspect, there is provided a method of providing a wellhead tree for a subsea hydrocarbon extraction facility with means for monitoring a plurality of different properties relating to hydrocarbon fluid flow through a pipeline at the wellhead tree, the method comprising providing a plurality of sensing means, each for monitoring at least one of said properties, and locating each of the sensing means at or near a position which is optimum for monitoring the at least one of the properties having regard to the configuration of the pipeline, characterised in that: one of said sensing means comprises means for monitoring vibration and/or strain, the method comprising locating it at or near a valve; and one of said sensing means comprises means for monitoring bulk density, the method comprising locating it at or near a point of turbulence in the pipeline.

According to the present invention from another aspect, there is provided a wellhead tree for a subsea hydrocarbon extraction facility, the wellhead tree being provided with means for monitoring a plurality of different properties relating to hydrocarbon fluid flow through a pipeline at the wellhead tree, said monitoring means comprising a plurality of sensing means, each for monitoring at least one of said properties, and each of the sensing means being located at or near a position which is optimum for monitoring the at least one of the properties having regard to the configuration of the pipeline, characterised in that: one of said sensing means comprises means for monitoring vibration and/or strain and is located at or near a valve; and one of said sensing means comprises means for monitoring bulk density and is located at or near a point of turbulence in the pipeline.

One of said sensing means could comprise means for monitoring vibration and/or strain and be located at or near a known weak point of the pipeline.

One of said sensing means could comprise means for monitoring particulates in said flow and be located at or near a bend in the pipeline.

One of said sensing means could be located at or near a region of steady state flow in the pipeline.

One of said sensing means could comprise means for monitoring erosion of the pipeline and be located at or near a position of most serious erosion of the pipeline.

One of said sensing means could comprise means for monitoring a pressure drop and be located at or near a restriction or known change in the geometry of the pipeline.

One of said sensing means could comprise means for monitoring temperature and be located at or near a region isolated from an interfering temperature.

### Brief Description of the Drawing

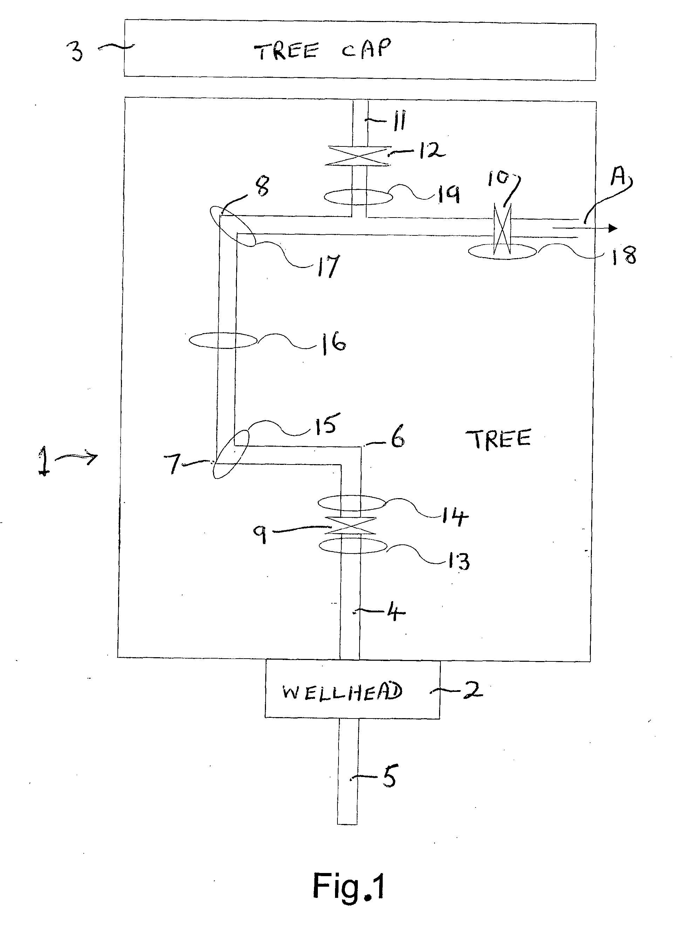

Fig. 1 is a simplified schematic illustration of a subsea Christmas tree according to the invention.

### Description of an Embodiment of the Invention.

A subsea Christmas tree of a subsea hydrocarbon extraction facility, in its basic form houses a number of valves for controlling the flow of fluid from a well (such as the main flow control and directional flow valves) together with a subsea control module which enables the valves to be controlled by means of electric or hydraulic actuators. There is also a sensor pack which provides essential data on the state of health of the subsea system and for the provision of data for optimising the fluid flow from the well.

The physical configuration of the Christmas tree mechanical structure and of the equipment installed results in the flow pipeline which carries the hydrocarbon fluid having several sharp bends, and use can be made of these to provide the necessary optimised positions for some sensor measurements.

Fig. 1 is a simplified schematic illustration showing the main hydrocarbon flow pipeline components and appropriate positions for installing some typical sensors in a tree according to an embodiment of the invention.

In Fig. 1, a subsea Christmas tree 1 at a wellhead 2 has a tree cap 3 and a flow pipeline 4 fed from production tubing 5. The flow pipeline 4 exits the tree 1 to a flow line in the direction of arrow A and has first, second and third severe or sharp bends 6, 7 and 8. Between the tubing 5 and bend 6, the pipeline 4 has a flow control valve 9 and between bend 8 and the flow line the pipe has a directional control valve 10, there being between bend 8 and valve 10 a branch section 11 of pipeline 4, having a directional control valve 12.

The embodiment of the invention utilises the knowledge of the flow regimes in the hydrocarbon flow pipeline and valve configurations on the tree to place suitable discrete sensors in the most appropriate positions to get a more accurate overall monitoring of properties relating to hydrocarbon fluid flow. The arrangement of sensors utilises the physical configuration of the tree, having regard to the configuration of the pipeline 4, to enable measurements of such properties to be made by using discrete sensors each placed at or near an optimum position in the fluid flow for its measurement in a most meaningful manner.

Typical measurements for which optimum positions (shown in Fig. 1) can be identified on the Christmas tree are:
- vibration and/or strain measurement - by locating a sensor 13 at or near valve 9, which could cause vibration, or at a known weak point;
- bulk density measurement - by locating a sensor 14 at or near a point of high turbulence such as after valve 9 or other disruption;
- particulate detection, such as sand detection - by locating an acoustic sensor 15 at or near severe bend 7 in pipeline 4 to detect particle impact, this being non-intrusive and fitted to the outside of the pipeline 4;
- ultrasound, electrical impedance spectroscopy, microwave measurements or similar - by locating a sensor 16 between bends 7 and 8 where there is a conditioned steady state flow;
- erosion measurement - by locating a sensor 17 at or near bend 8 where there is most serious erosion, to make a direct measurement;
- pressure drop - by locating a sensor 18 which measures pressure drop through a restriction or known change in geometry, such as the pressure drop across valve 10; and
- temperature measurement - by placing a sensor 19 before valve 12 at or near a most isolated point from any interfering temperature.

### Advantages of using the Invention

Technical:
- It offers a significantly more detailed and accurate method of measuring produced fluid properties compared to conventional methods

Commercial:
- It would offer increased functionality for an Christmas tree compared to the conventional approach of integrating instruments attached to a tree as stand-alone instrument packages.

## Claims

1. A method of monitoring hydrocarbon fluid flow of a subsea hydrocarbon extraction facility with means for monitoring a plurality of different properties relating to the hydrocarbon fluid flow through a pipeline (4) at a wellhead tree, the method comprising providing a plurality of sensing means (13, 14, 15, 16, 17, 18, 19), each for monitoring at least one of said properties, and locating each of the sensing means at or near a position which is optimum for monitoring the at least one of the properties having regard to the configuration of the pipeline, **characterised in that**: one of said sensing means comprises means (13) for monitoring vibration and/or strain, the method comprising locating it at or near a valve (9); and one of said sensing means (14) comprises means for monitoring bulk density, the method comprising locating it at or near a point of turbulence in the pipeline.

2. A method according to claim 1, wherein one of said sensing means comprises means (13) for monitoring vibration and/or strain, the method comprising locating it at or near a known weak point of the pipeline (4).

3. A method according to any preceding claim, wherein one of said sensing means comprises means (15) for monitoring particulates in said flow, the method comprising locating it at or near a bend (7) in the pipeline (4).

4. A method according to any preceding claim, comprising locating one of said sensing means (16) at or near a region of steady state flow in the pipeline (4).

5. A method according to any preceding claim, wherein one of said sensing means comprises means (17) for monitoring erosion of the pipeline (4), the method comprising locating it at or near a position of most serious erosion of the pipeline.

6. A method according to any preceding claim, wherein one of said sensing means comprises means (18) for monitoring a pressure drop, the method comprising locating it at or near a restriction or known change in the geometry of the pipeline (4).

7. A method according to any preceding claim, wherein one of said sensing means comprises means (19) for monitoring temperature, the method comprising locating it at or near a region isolated from an interfering temperature.

8. A wellhead tree (1) for a subsea hydrocarbon extraction facility, the tree being provided with means for monitoring a plurality of different properties relating to hydrocarbon fluid flow through a pipeline (4) at the tree, said monitoring means comprising a plurality of sensing means (13, 14, 15, 16, 17, 18, 19), each for monitoring at least one of said properties, and each of the sensing means being located at or near a position which is optimum for monitoring the at least one of the properties having regard to the configuration of the pipeline, **characterised in that**: one of said sensing means comprises means (13) for monitoring vibration and/or strain and is located at or near a valve (4); and one of said sensing means comprises means (14) for monitoring bulk density and is located at or near a point of turbulence in the pipeline.

9. A tree (1) according to claim 8, wherein one of said sensing means comprises means (13) for monitoring vibration and/or strain and is located at or near a known weak point of the pipeline (4).

10. A tree (1) according to claim 8 or 9, wherein one of said sensing means comprises means (15) for monitoring particulates in said flow and is located at or near a bend (7) in the pipeline (4).

11. A tree (1) according to any of claims 8 to 10, wherein one of said sensing means (16) is located at or near a region of steady state flow in the pipeline (4).

12. A tree (1) according to any of claims 8 to 11, wherein one of said sensing means comprises means (17) for monitoring erosion of the pipeline (4) and is located at or near a position of most serious erosion of the pipeline.

13. A tree (1) according to any of claims 8 to 12, wherein one of said sensing means comprises means (18) for monitoring a pressure drop and is located at or near a restriction or known change in the geometry of the pipeline (4).

14. A tree (1) according to any of claims 8 to 13, wherein one of said sensing means comprises means (19) for monitoring temperature and is located at or near a region isolated from an interfering temperature.

## Patentansprüche

1. Verfahren zum Überwachen eines Kohlenwasserstoff-Fluidstroms einer Untersee-Kohlenwasserstoff-Gewinnungseinrichtung mit Mitteln zum Überwachen mehrerer verschiedener, den Kohlenwasserstoff-Fluidstrom durch eine Rohrleitung (4) an einem Bohrlochkopf-Eruptionskreuz (4) betreffender Eigenschaften, wobei das Verfahren das Bereitstellen mehrerer Abfühlmittel (13, 14, 15, 16, 17, 18, 19), die jeweils zum Überwachen von mindestens einer der Eigenschaften dienen, und das Positionieren von jedem der Abfühlmittel an oder nah bei einem Ort, der unter Berücksichtigung der Konfiguration der Rohrleitung zum Überwachen der mindestens einen der Eigenschaften optimal ist, umfasst, **dadurch gekennzeichnet, dass**: eines der Abfühlmittel Mittel (13) zum Überwachen von Schwingung und/oder Belastung umfasst, wobei das Verfahren das Positionieren davon an oder nah bei einem Ventil (9) umfasst; und eines der Abfühlmittel (14) Mittel zum Überwachen der Rohdichte umfasst, wobei das Verfahren das Positionieren davon an oder nah bei einer Stelle mit Turbulenzen in der Rohrleitung umfasst.

2. Verfahren nach Anspruch 1, wobei eines der Abfühlmittel Mittel (13) zum Überwachen von Schwingungen und/oder Belastung umfasst, wobei das Verfahren das Positionieren davon an oder nah bei einer bekannten Schwachstelle der Rohrleitung (4) umfasst.

3. Verfahren nach einem der vorangehenden Ansprüche, wobei eines der Abfühlmittel Mittel (15) zum Überwachen von Schwebstoffen in dem Strom umfasst, wobei das Verfahren das Positionieren davon an oder nah bei einem Bogen (7) in der Rohrleitung (4) umfasst.

4. Verfahren nach einem der vorangehenden Ansprüche, umfassend das Positionieren eines der Abfühlmittel (16) an oder nah bei einem Bereich stationärer Strömung in der Rohrleitung (4).

5. Verfahren nach einem der vorangehenden Ansprüche, wobei eines der Abfühlmittel Mittel (17) zum Überwachen der Erosion der Rohrleitung (4) umfasst, wobei das Verfahren das Positionieren davon an oder nah bei einem Ort der schwerwiegendsten Erosion der Rohrleitung umfasst.

6. Verfahren nach einem der vorangehenden Ansprüche, wobei eines der Abfühlmittel Mittel (18) zum Überwachen eines Druckabfalls umfasst, wobei das Verfahren das Positionieren davon an oder nah bei einer Einengung oder bekannten Änderung der Geometrie der Rohrleitung (4) umfasst.

7. Verfahren nach einem der vorangehenden Ansprüche, wobei eines der Abfühlmittel Mittel (19) zum Überwachen der Temperatur umfasst, wobei das Verfahren das Positionieren davon an oder nah bei einem von einer störenden Temperatur isolierten Bereich umfasst.

8. Bohrlochkopf-Eruptionskreuz (1) für eine Untersee-Kohlenwasserstoff-Gewinnungseinrichtung, wobei das Eruptionskreuz mit Mitteln zum Überwachen mehrerer verschiedener, den Kohlenwasserstoff-Fluidstrom durch eine Rohrleitung (4) an dem Eruptionskreuz betreffender Eigenschaften versehen ist, wobei die Überwachungsmittel mehrere Abfühlmittel (13, 14, 15, 16, 17, 18, 19) umfassen, die jeweils zum Überwachen mindestens einer der Eigenschaften dienen, und sich jedes der Abfühlmittel an oder nah bei einem Ort befindet, der unter Berücksichtigung der Konfiguration der Rohrleitung für die Überwachung der mindestens einen der Eigenschaften optimal ist, **dadurch gekennzeichnet, dass**: eines der Abfühlmittel Mittel (13) zum Überwachen von Schwingung und/oder Belastung umfasst, und sich an oder nah bei einem Ventil (4) befindet; und eines der Abfühlmittel Mittel (14) zum Überwachen der Rohdichte umfasst und sich an oder nah bei einer Stelle mit Turbulenzen in der Rohrleitung befindet.

9. Eruptionskreuz (1) nach Anspruch 8, wobei eines der Abfühlmittel Mittel (13) zum Überwachen von Schwingung und/oder Belastung umfasst und sich an oder nah bei einer bekannten Schwachstelle der Rohrleitung (4) umfasst.

10. Eruptionskreuz (1) nach Anspruch 8 oder 9, wobei eines der Abfühlmittel Mittel (15) zum Überwachen von Schwebstoffen in dem Strom umfasst und sich an oder nah bei einem Bogen (7) in der Rohrleitung (4) befindet.

11. Eruptionskreuz (1) nach einem der Ansprüche 8 bis 10, wobei sich eines der Abfühlmittel (16) an oder nah bei einem Bereich stationärer Strömung in der Rohrleitung (4) befindet.

12. Eruptionskreuz (1) nach einem der Ansprüche 8 bis 11, wobei eines der Abfühlmittel Mittel (17) zum Überwachen der Erosion der Rohrleitung (4) umfasst und sich an oder nah bei einem Ort der schwerwiegendsten Erosion der Rohrleitung umfasst.

13. Eruptionskreuz (1) nach einem der Ansprüche 8 bis 12, wobei eines der Abfühlmittel Mittel (18) zum Überwachen eines Druckabfalls umfasst und sich an oder nah bei einer Einengung oder bekannten Änderung der Geometrie der Rohrleitung (4) befindet.

14. Eruptionskreuz (1) nach einem der Ansprüche 8 bis 13, wobei eines der Abfühlmittel Mittel (19) zum Überwachen der Temperatur umfasst und sich an oder nah bei einem von einer störenden Temperatur isolierten Bereich befindet.

## Revendications

1. Procédé de surveillance d'un écoulement de fluide d'hydrocarbures d'une installation d'extraction d'hydrocarbures sous-marine avec des moyens de surveillance d'une pluralité de propriétés différentes concernant l'écoulement de fluide d'hydrocarbures dans un pipeline (4) au niveau d'un arbre de tête de puits, le procédé comprenant la fourniture d'une pluralité de moyens de détection (13, 14, 15, 16, 17, 18, 19), chacun servant à surveiller au moins l'une desdites propriétés, et le positionnement de chacun des moyens de détection à une position optimale ou près d'une position optimale pour la surveillance d'au moins l'une des propriétés en fonction de la configuration du pipeline, **caractérisé en ce que** : l'un desdits moyens de détection comprend un moyen (13) pour surveiller une vibration et/ou une contrainte, le procédé comprenant son placement au niveau, ou près, d'une vanne (9) ; et l'un desdits moyens de détection (14) comprend un moyen pour surveiller une densité apparente, le procédé comprenant son positionnement à un point de turbulence dans le pipeline, ou près de ce point.

2. Procédé selon la revendication 1, dans lequel l'un desdits moyens de détection comprend un moyen (13) pour surveiller une vibration et/ou une contrainte, le procédé comprenant son positionnement à un point de faiblesse connu du pipeline (4) ou près de ce point.

3. Procédé selon l'une quelconque des revendications précédentes, dans lequel l'un desdits moyens de détection comprend un moyen (15) pour surveiller des particules dans ledit écoulement, le procédé comprenant son positionnement au niveau d'un coude (7) dans le pipeline (4) ou près de ce coude.

4. Procédé selon l'une quelconque des revendications précédentes, comprenant le positionnement de l'un desdits moyens de détection (16) au niveau d'une région d'écoulement régulier dans le pipeline (4) ou près de celle-ci.

5. Procédé selon l'une quelconque des revendications précédentes, dans lequel l'un desdits moyens de détection comprend un moyen (17) pour surveiller l'érosion du pipeline (4), le procédé comprenant son positionnement à une position d'érosion critique du pipeline ou près de celle-ci.

6. Procédé selon l'une quelconque des revendications précédentes, dans lequel l'un desdits moyens de détection comprend un moyen (18) pour surveiller une chute de pression, le procédé comprenant son positionnement au niveau d'une restriction ou d'un changement connu de la géométrie du pipeline (4) ou près de cette restriction ou de ce changement.

7. Procédé selon l'une quelconque des revendications précédentes, dans lequel l'un desdits moyens de détection comprend un moyen (19) pour surveiller la température, le procédé comprenant son positionnement dans une région isolée d'une température parasite ou près de cette région.

8. Arbre de tête de puits (1) d'une installation d'extraction d'hydrocarbures sous-marine, l'arbre étant doté de moyens de surveillance d'une pluralité de propriétés différentes concernant l'écoulement de fluide d'hydrocarbures dans un pipeline (4) au niveau de l'arbre, lesdits moyens de surveillance comprenant une pluralité de moyens de détection (13, 14, 15, 16, 17, 18, 19), chacun servant à surveiller au moins l'une desdites propriétés, et chacun des moyens de détection étant positionné à une position optimale ou près d'une position optimale pour la surveillance d'au moins l'une des propriétés en fonction de la configuration du pipeline, **caractérisé en ce que** : l'un desdits moyens de détection comprend un moyen (13) pour surveiller une vibration et/ou une contrainte, et est situé au niveau, ou près, d'une vanne (4) ; et l'un desdits moyens de détection comprend un moyen (14) pour surveiller la densité apparente et est positionné à un point de turbulence dans le pipeline, ou près de ce point.

9. Arbre (1) selon la revendication 8, dans lequel l'un desdits moyens de détection comprend un moyen (13) pour surveiller une vibration et/ou une contrainte et est positionné à un point de faiblesse connu du pipeline (4) ou près de ce point.

10. Arbre (1) selon la revendication 8 ou 9, dans lequel l'un desdits moyens de détection comprend un moyen (15) pour surveiller des particules dans ledit écoulement et est positionné au niveau d'un coude (7) dans le pipeline (4) ou près de ce coude.

11. Arbre (1) selon l'une quelconque des revendications 8 à 10, dans lequel l'un desdits moyens de détection (16) est positionné au niveau d'une région d'écoulement régulier dans le pipeline (4) ou près de celle-ci.

12. Arbre (1) selon l'une quelconque des revendications 8 à 11, dans lequel l'un desdits moyens de détection comprend un moyen (17) pour surveiller l'érosion du pipeline (4) et est positionné à une position d'érosion critique du pipeline ou près de celle-ci.

13. Arbre (1) selon l'une quelconque des revendications 8 à 12, dans lequel l'un desdits moyens de détection comprend un moyen (18) pour surveiller une chute de pression et est positionné au niveau d'une restriction ou d'un changement connu de la géométrie du pipeline (4) ou près de cette région ou de ce changement.

14. Arbre (1) selon l'une quelconque des revendications 8 à 13, dans lequel l'un desdits moyens de détection comprend un moyen (19) pour surveiller la température et est positionné dans une région isolée d'une température parasite ou près de cette région.
